# EUROPEAN PATENT APPLICATION

(11) **EP 4 230 199 A1**
(43) Date of publication of application: **23.08.2023**
(21) Application number: 20964395.6
(22) Date of filing: 04.12.2020
(51) Int. Cl.: A61K 31/197, A61K 31/137, A61P 25/04

(54) **STABLE COATED SOLID PHARMACEUTICAL COMPOSITION OF AN OPIOID ANALGESIC AND AN ANTI-EPILEPTIC FOR PAIN**

(71) Applicant: Laboratorios Silanes, S.A. de C.V., Mexico, 11000 (MX)
(72) Inventor: MUÑOZ MARTÍNEZ, Cecilia Jannette, Ciudad de México, 11000 (MX); GONZÁLEZ CANUDAS, Jorge Alejandro, Ciudad de México, 11000 (MX); OLLERVIDES RUBIO, Paola Yazmín, Ciudad de México, 11000 (MX); ESPINOZA LEÓN, Sixto Serafín, Ciudad de México, 11000 (MX); CUAHUTENCOS ESCOBAR, Ernesto, Ciudad de México, 11000 (MX)
(74) Representative: Araujo, Daniel
(86) International application number: PCT/MX2020/050050
(87) International publication number: WO 2022/119430

(57) **Abstract**

This invention comprises pharmaceutical compositions consisting of tramadol and pregabalin, or a pharmaceutically acceptable salt thereof, for the treatment of neuropathic pain. Besides offering an analgesic effect and a decrease of adverse events due to the reduced dose of one or both compounds, it has an improved stability, thus maintaining the dissolution and bioavailability of the composition to be administered. The composition manages to overcome the technological difficulty of having two humidity- and light-sensitive drugs in a dispensing medium that does not affect their absorption by means of a water-free process and a light protection coating without affecting the release of both drugs.

## Description

### FIELD OF THE INVENTION

This invention refers to pharmaceutical compositions aimed to treat and manage pain.

### BACKGROUND OF THE INVENTION

According to the International Association for the Study of Pain (IASP), a neuropathic pain is a result of an injury or a disease affecting the somatosensory system.

It is known that patients suffering from an acute neuropathic pain do not always respond to treatments or may develop resistance to the current drug products.

Despite the availability of many effective drugs and the guidelines on treating neuropathic pain, the American and European tests suggest that they are not widely used, and many cases remain untreated or with no treatment (IASP).

For instance, the formulation I of tramadol, the nomenclature of which is (1R,2R)-2-[(dimethylamino)methyl]-1-(3-methoxyphenyl)cyclohexan-1-ol, is a synthetic codeine analog and a centrally-acting analgesic containing opioid agonists properties that activate the monoaminergic spinal inhibition of pain, and is commonly used for treating acute neuropathic pains.

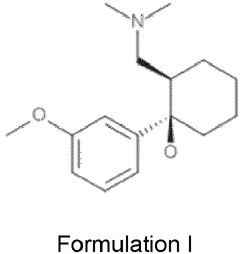

It can be administered orally, rectally, intravenously, or intramuscularly. In patients suffering from moderate to severe postoperative pain, intravenous or intramuscular tramadol has proven to have a potency equivalent to pethidine (meperidine) and to be one-fifth as potent as nalbuphine. Tramadol has shown analgesic activity in different animal models and in healthy volunteers with experimentally-induced pain; oral tramadol possesses a similar analgesic activity (Lee, Rhoda; McTavish, Donna; Sorkin, Eugene; Tramadol; A Preliminary Review of its Pharmacodynamic and Pharmacokinetic Properties, and Therapeutic Potential in Acute and Chronic Pain States, 1993).

Common side effects from tramadol administration may include constipation, nausea, vomiting, stomachache, dizziness, drowsiness, tiredness and headache. Some side effects may be severe, such as convulsions; hives; blisters; difficulty swallowing; swelling of the eyes, face, throat, tongue, lips, hands, feet, ankles; and even changes in heart rate. Moreover, tramadol may cause addiction, especially after long-term use. It is known that high doses of tramadol may result in severe side effects. Intentional and accidental overdoses of tramadol may cause respiratory arrests, as well as acute liver failure; several fatal cases have been reported. However, in these instances, liver injuries may be a result of shock, hypoxia or ischemia secondary to respiratory arrest. Liver injury attributed to tramadol overdose has also been associated to hyperammonemia, lactic acidosis, and hepatic steatosis. This suggests a direct mitochondrial injury (Liver Tox Clinical and Research information of Drug-Induce Livered Injury, National Institutes of Health, December 05, 2012).

Alternatively, there is another drug called pregabalin, also known as (S)-3-(aminomethyl)-5-methylhexanoic acid, formulation II. It is a neuromodulator drug with a better pharmacokinetic profile than its predecessors; it is also a GABA analog, although it does not perform all GABAergic actions. Pregabalin has a better affinity than gabapentin, as well as an analgesic effect, due to its ability to bind to the alpha-2-delta protein subunit of voltage-gated calcium channels in the Central Nervous

System (González Escalada, R.L; Pregabalin in the treatment of peripheral neuropathic pain, Revista de la Sociedad Española para el dolor, 2005).

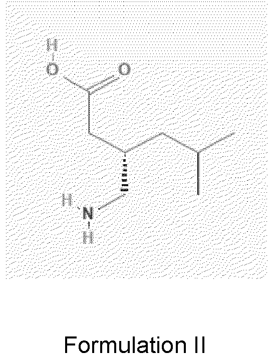

Pregabalin can have side effects, such as tiredness, dizziness, headache, dry mouth, nausea, vomiting, constipation, abdominal distension, speech problems, anxiety, loss of balance, muscular spams, weakness, among others. Some of these effects may be severe, such as blurred or double vision; hives; blisters; swelling of the face; swelling of the arms, hands, and/or feet; shortness of breath; muscle pain; and chest pain. Adverse effects caused by the high doses of pregabalin include dizziness, drowsiness, peripheral edema, dry mouth, headache, confusion, depression, and visual disturbances.

To avoid the most common adverse effects and those produced by high doses of tramadol or pregabalin, many drug combinations that produce an analgesic effect without increasing the dose have been developed. For instance, the patent application with international publication number WO 2002/091990 provides a composition to treat pain. The composition includes a pharmaceutically-acceptable analgesic with a GABAergic agent, such as gamma vinyl GABA, which is effective to reduce or inhibit the collateral effect of the addictive load of the analgesic.

Said document does not indicate nor describe a synergic effect of the combination of an analgesic with a GAB agent. More recently, the Mexican application MX/a/2013/000024 refers to the extended-release combination of an opioid and a GABA analog in different pharmaceutical layers to improve the absorption within the body during pain treatment. The patent application with international publication number WO2007052999 describes a composition to manage pain comprising of a mix of an analgesic (tramadol) and an antiepileptic (gabapentin) at concentrations of 3-8.3% y 16-83%, respectively. Gabapentin is slowly absorbed, shows saturation peaks, and its dissolution profiles are not linear, unlike pregabalin, which is rapidly absorbed by the blood.

Other examples of combinations include patent number US2013189354, which describes the composition in gelatin capsules containing tramadol, pregabalin, and dextromethorphan at low concentrations. However, this composition does not describe nor indicate a synergy between the compounds. Furthermore, patent application publication number US2015313892 describes the combination of tramadol with gabapentin or pregabalin, the latter at concentrations of 15 to 30 mg. Although this combination is described as synergic, testing on such synergy is not available. Mexican patent application MX2017016720 describes the combination of an γ-aminobutyric acid (Gaba) with an opioid analgesic drug, in which the former is pregabalin and the latter is tramadol. However, said application does not claim nor describe how to overcome the stability challenge and the combination of both drugs without impacting the bioavailability and dissolution of both compounds. On the other hand, document WO220044140 details a synergic pharmaceutical combination containing tramadol hydrochloride and pregabalin in a p/p ratio of 1:1.5 and 1 :2.5. Their excipients are pharmaceutically applicable, since the dosage forms of said synergic form are oral and parenteral. This invention also implies methods to relief neuropathic pain, such as central or peripheral pain, among others, as well as the synergic pharmaceutical combination to be used in neuropathic pain treatments. On one hand, this document does not address the pharmacotechnical issue of combining these active ingredients in a stable manner without affecting the stability, quality, and efficacy parameters.

On the other hand, the publication named "Pregabalin antinociception and its interaction with tramadol in acute model of pain" shows the antinociceptive study on pregabalin and tramadol.

The publication concludes that both drugs possess similar antinoniceptive effects but, when they are administered as a combined treatment, there is a superadditive interaction.

The article "Efficacy, Safety, Tolerability and Pharmacokinetics of Concomitant Administration of Tramadol With Duloxetine or Pregabalin: a Randomized Controlled Flexible-dose Study in Patients With Neuropathic Pain" compares, on a double-blind study, the combination of tramadol with duloxetine (30 mg/75 mg dose) or tramadol with pregabalin (30 mg/75 mg dose) vs. placebo and tramadol using an initial daily dose of 100 mg; every drug is administered separately.

However, there are other issues related to the development of formulations associated with these types of drugs that relieve pain. The European patent EP2343055 describes the formulation issues related to pregabalin due to the presence of external factors, such as humidity. This is resolved by adding high amounts of colloid silicon dioxide. The patent's preferred method of formulation is capsules, due to the many of stages involved in the tablet formulation. Nevertheless, this patent does neither detail nor point out the formulation of a second drug such as tramadol or how to solve the problem of administering high concentration drugs due to the limited size of the capsules without affecting, at the same time, their dissolution, bioavailability, and stability. The patent document WO2008128775 claims a composition free of sacharides and lactose without containing more aminoacids. This composition is not formulated with water. This document does not mention the use of a special coating system; therefore, different issues may arise during storage due to the lack of protection from external factors, such as light, temperature, humidity, etc. Consequently, its release may be affected. Patent application AU2017300185 claims a pregabalin composition of extended release with a special coating system. The Mexican patent MX276428 also mentions the addition of a coating component or gelling agent that may delay the drug release.

In addition, publication WO2006078811 seeks to protect a three-component coating system that provides a prolonged release.

The above-mentioned patents make use of different coating systems that affect the drug release but do not solve the issues that impact the drug's organoleptic properties, such as its stability, dissolution, and bioavailability.

The current state of the art also does not solve the problem of finding a combination of both drugs for the treatment and management of pain that, in addition to maintaining low doses, achieves a synergistic effect, therefore improving bioavailability, dissolution, stability and, at the same time, the reduction of side effects once the combination is administered to the patient.

The lack of solutions and other options to the above-mentioned issues causes the therapeutic effect to be delayed or higher doses to be required. In addition to the problems of synergy and increased side effects due to the conformation of high doses of tramadol and pregabalin, there is the technical problem of combining two drugs with high sensitivity to light and humidity.

Thus, this invention involves stable, immediate-release combination pharmaceutical compositions with a synergistic effect that maintain dissolution and bioavailability in the scope of pain treatment and management.

### SUMMARY OF THE INVENTION

This invention comprises pharmaceutical compositions consisting of tramadol and pregabalin, or a pharmaceutically acceptable salt thereof, for the treatment of neuropathic pain and, preferably, acute neuropathic pain. Besides offering an analgesic effect and a decrease of adverse events due to the reduced dose of one or both compounds, it has an improved stability, thus maintaining the dissolution and bioavailability of the composition to be administered.

One form of the invention comprises a single-dose composition of tramadol hydrochloride at a concentration of 50 to 150 mg and 50 to 150 mg of pregabalin, preferably 50-100 mg and 75-150 mg respectively.

In another form of the invention, the pharmaceutical composition is characterized by being in the form of a tablet, pill, caplet, granules, or capsules. It is preferably in a tablet and/or pill form.

In another form of the invention, the most preferred dosage form is a tablet or, otherwise, a caplet with a breakline in its design.

Furthermore, the excipients comprising the composition that provide the invention with essential characteristics that improve the pharmacokinetic profile, bioavailability, stability, and dissolution are also the object of this invention.

Another form of the invention deals with the manufacture process of the composition to overcome the technological complexity of having two drugs, which are sensitive to humidity and light, in a dispensing medium that does not affect their absorption by means of a process that incorporates water as a solvent, since there is minimal contact with the surface of the tablet; in addition, the composition is coated to protect it against light, so that the release of both drugs is not affected.

### BRIEF FIGURES DESCRIPTION

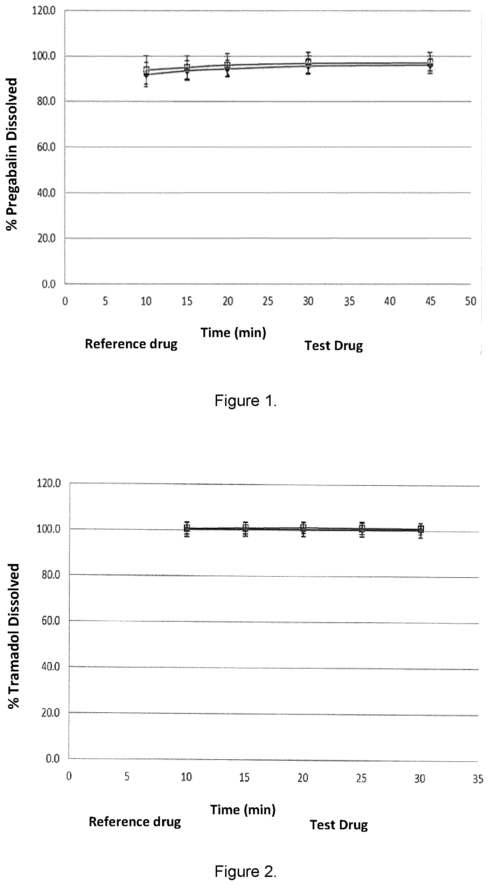

Figure 1. Dissolved Pregabalin; comparison of the dissolution profile of the reference medicine [50 mg tramadol hydrochloride/150 mg pregabalin] with that of the test medicine [50 mg tramadol hydrochloride/75 mg pregabalin]; both batches from Laboratorios Silanes S.A. de CV. The maximum difference between the dissolution percentages of the medicine is less than or equal to 2.0% within the sampling times.
**Figure 2****:** Dissolved Tramadol; comparison of the dissolution profile of the reference medicine [50 mg tramadol hydrochloride/150 mg pregabalin] with that of the test medicine [50 mg tramadol hydrochloride/75 mg pregabalin]; both batches from Laboratorios Silanes S.A. de CV.

### DETAILED DESCRIPTION OF THE INVENTION

### Definitions

Pharmaceutically acceptable salt: The term "pharmaceutically acceptable salt" of a given compound refers to the salts that hold the biological efficacy of said compound, as well as its properties. These salts are not biologically or otherwise undesirable (P. Heinrich Stahl and Camille G. Wermuth (Eds.) Pharmaceutical Salts Properties, Selection, and Use (International Union of Pure and Applied Chemistry), Wiley-VCH; 2a Revised Edition (May 16, 2011)). Pharmaceutically acceptable base addition salts can be prepared from inorganic or organic bases. Some examples of salts deriving from inorganic bases include sodium, potassium, lithium, ammonium, calcium, and magnesium salts. Some examples of salts deriving from organic bases include primary, secondary, and tertiary amine salts. Specific examples of adequate amines include isopropylamine, trimethylamine, diethylamine, tri(isopropyl)amine, tri(n-propyl)amine, ethanolamine, 2-dimethylaminoethanol, tromethamine, lysine, arginine, histidine, caffeine, procaine, hydrabamine, choline, betaine, ethylenediamine, glucosamine, N- alkylglucamines, theobromine, purines, piperazine, piperidine, morpholine, N-ethylpiperidine, and similar.

Pharmaceutically acceptable base addition salts can be prepared from inorganic or organic acids. Salts deriving from inorganic acids include hydrochloric acid, hydrobromic acid, sulfuric acid, nitric acid, phosphoric acid, and similar. Salts deriving from organic acids include acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methanesulfonic acid, ethanesulfonic acid, p-toluenesulfonic acid, salicylic acid, and similar.

For instance, there are two pregabalin salts derived from sulfuric acid and described in the patent document WO2009080365A1 "Pregabalin salts" that are synthesized: pregabalin besylate and tosylate, assuming that the acid anion is not toxic to humans. Other prepared salts were pregabalin hydrochloride (WO 2005/041927) and pregabalin mandelate (WO 96/40617).

Pharmaceutical compositions: the composition of this invention may include any pharmaceutically acceptable form of pregabalin and/or tramadol, along with its pharmaceutically acceptable complexes, salts, solvates, hydrates, and polymorphs.

Neuropathic pain: according to the International Association for the Study of Pain (IASP), a neuropathic pain is a direct result of an injury or a disease affecting the somatosensory system.

This type of pain is present in several neuropathies, polyneuropathies, post-therapeutic neuralgia, other common central pain syndromes, e.g., spinal cord injury, spinal cord tumor, syringomyelia, or cancer associated with neuropathic pain.

Excipient: it is the ingredient that are part of this pharmaceutical composition including, among others, diluents, disintegrants, lubricants, coating systems, and absorbents.

This invention relates to stable, immediate-release pharmaceutical compositions, with synergistic effect and improved stability, while maintaining dissolution and bioavailability of an opioid analgesic and an antiepileptic/analgesic, which can be administered as a therapeutic agent to treat pain. The pain may be neuropathic and, preferably, acute neuropathic pain.

The opioid analgesic used is tramadol or a pharmaceutically acceptable salt thereof, such as its hydrochloride salt, with an antiepileptic/analgesic, which is pregabalin, or a pharmaceutically acceptable salt thereof.

The combination of both drugs tramadol and pregabalin suggests an antinociceptive effect in animal models and a decrease in tramadol adverse events, such as seizures. (2012, Fariborz; 2015, Tewari). The combination of these drugs offers the possibility of an efficient analgesia and a decrease in adverse events reported with single drugs due to the reduced doses of one or both compounds (2017, Suthakaran).

The combination of tramadol and pregabalin represents a set of important technological challenges due to the physicochemical properties of the drugs. The proper selection of excipients and the manufacturing conditions play a very important role in the development of pharmaceutical compositions in relation to the release of the drug and the absorption rate in the organism. That way, the composition overcomes the technological complexity of combining both drugs, which are sensitive to humidity and light, in a dispensing medium that does not affect their absorption by means of a process that incorporates water as a solvent, since there is minimal contact with the surface of the tablet; in addition, the composition is coated to protect it against light, so that the release of both drugs is not affected and stability is improved.

This invention includes the combination of tramadol and pregabalin or a pharmaceutically acceptable salt thereof in doses of 50 to 150 mg of tramadol and 50 to 150 mg of pregabalin -preferably in low doses, such as 50 to 100 mg of tramadol or any of its pharmaceutically acceptable salts, and 75 to 150 mg of pregabalin or any of its pharmaceutically acceptable salts at concentrations- with at least one pharmaceutically acceptable excipient.

The solid pharmaceutical composition can be found as a tablet, monolayer tablet, granules, caplets, lozenges, or pills. It is preferably in a tablet and/or pill form.

The most preferred dosage form of the invention is "tablets" because of its dosage accuracy. It is also the most widely accepted dosage form as it is easy to administer, making it possible to dose a high concentration of drugs, as opposed to capsules. The volume of the powders in tablets can be reduced, thus facilitating their handling and administration.

In a form of the invention, the preferred dosage form is a tablet or, otherwise, a caplet with a breakline in its design.

In another form of the invention, the most preferred dosage form is a biconvex tablet.

Examples of pharmaceutically acceptable diluents include, among others, cellulose derivatives such as microcrystalline cellulose PH102, phosphate derivatives such as dibasic calcium phosphate, starch derivatives such as pregelatinized starch and corn starch, as well as mannitol, xylitol, maltitol, lactitol, sorbitol, sucrose, or a combination thereof. The preferred diluent for this invention is the microcrystalline cellulose PH102. This diluent should be preferably present at a concentration ranging from 50 to 90 wt%.

Pharmaceutically acceptable disintegrants include, among others, croscarmellose, cellulose derivatives such as hydroxypropyl cellulose, carboxymethyl cellulose, or microcrystalline cellulose; povidone derivatives such as crospovidone; starch derivatives such as pregelatinized starch or sodium starch glycolate; and corn starch. The disintegrant must be present in an amount ranging from 0.5 to 15 wt%, preferably between 0.5 to 3 wt%. The preferred disintegrant for this formulation is sodium croscarmellose.

Examples of the pharmaceutically acceptable lubricants include, among others, magnesium stearate, zinc stearate, calcium stearate, stearic acid, monostearate, stearyl fumarate, talc, and sulfated derivatives such as magnesium lauryl sulfate. The lubricant must be present in an amount ranging from 0.25 to 10%, preferably from 0.8 % to 1 wt%. The preferred lubricant for the composition of this invention is magnesium stearate.

Another form of the invention deals with the composition process to overcome the technological complexity of combining both drugs, which are sensitive to humidity and light, in a dispensing medium that does not affect their absorption, by means of a process that incorporates water as a solvent, since there is minimal contact with the surface of the tablet; in addition, the composition is coated to protect it against light, so that the release of both drugs is not affected and stability is improved. Tramadol and pregabalin are known to be sensitive to light and environmental humidity; therefore, to date, no drug product has been able to combine these two drugs in a single formulation without being affected by these factors. We were able to develop a process where drugs have the least contact with water. In order to achieve this, we selected a process to obtain the core (uncoated tablet) by using specific excipients and additives that maintain the chemical integrity of both drugs together, as well as the physical integrity of the tablet.

In this respect, and in another form of the invention, the production process of the tablets was based on the selection of the unit operations, as well as in the order and the execution time to control the different physicochemical properties of the drugs, which consisted in the selection of the correct coating ingredient and the appropriate temperatures to prevent drugs from having a very lengthy contact with water and to obtain, at the same time, a barrier against light.

Ideally, the coating system is selected from cellulose derivatives, such as hydroxypropyl methylcellulose; hydroxypropyl cellulose; carboxymethyl celluloses; polyvinyl derivatives, such as polyvinyl alcohol; polyethylene glycol; and povidones in all K values, as well as their derivatives. Hydroxypropyl methylcellulose is preferred at concentrations of 0.5 to 6 wt% and, most ideally, at concentrations of 2.5 to 3.5 wt%. Furthermore, the coating should preferably not use alcohol-based vehicles but rather be water.

In another part of the invention the solids concentration of the coating systems does not exceed 19.6%. This is important, since it ensures that the film is added to the final dosage form, so that the contact of the solvent with the surface of the solid dosage form or tablet is minimal.

Additionally, as absorbents of the residual water of the tablet, the composition of this invention comprises, among others, the following: aluminum derivatives (aluminum hydroxide, aluminum oxide, aluminum phosphate), clay or soil derivatives (attalpugite, bentonite, hectorite, kaolin, pectin), silica derivatives (calcium silicate, colloidal silicon dioxide, magnesium aluminum silicate), cellulose derivatives (microcrystalline cellulose, cellulose), and magnesium derivatives (magnesium carbonate, magnesium silicate). The preferred absorbent is the amorphous form of magnesium aluminum metasilicate in concentrations of 0.5 to 90 wt% and, most preferably, 1 to 1.5 wt%.

The additional advantage offered by the coating system of this invention as a tablet dosage form, preferably a caplet, is that it does not interfere with or delay the dissolution process of the drugs, as described in the dissolution test example.

The pharmaceutical composition of another form of the invention was not found to show pharmacokinetic interaction when the combined formulation of tramadol and pregabalin was administered.

For another form of the invention, the dissolution profiles of the aforementioned pharmaceutical combination include a comparison between the two concentrations to demonstrate biowaiver.

### Examples

### Example 1. Tablet manufacturing process

The pharmaceutical composition of the invention is manufactured based on the selection of the unit operations, as well as on the order and the execution time to control the different physicochemical properties of the drugs. The process consisted in selecting the correct coating ingredient and the ideal temperatures to avoid a very long water contact with the drugs and, thus, obtain a barrier against light. The unit operations include sieving, mixing, and compressing.

On the other hand, for any coating stage, the product temperature is essential to achieve adherence of the film to the core surface. In general, for this type of hydroxypropyl methylcellulose-based systems, temperatures of 40 °C to 45 °C are used. For one form of this invention, a coating process with a core temperature between 45-50°C was successfully implemented to adhere the film to the core surface and to reduce the contact of the water contained in the coating suspension with the core. This maintains the stability of the product during the coating process and provides protection against light at the end of the process.

Some of the steps of the tablet (in caplet dosage form) manufacturing process of this invention comprising tramadol and pregabalin and/or their pharmaceutically acceptable salts are provided below:
1. Mix 45% diluent, 50% adsorbent, and drug 1 for 3 minutes and sieve (sieved substance 1).
2. Mix the diluent, the remaining adsorbent, and the drug 2 for 3 minutes and sieve (sieved substance 2).
3. Mix the sieved substance 1, the sieved substance 2, and the disintegrant agent for 5 minutes (mix 3).
4. Sieve the lubricant and mix with the powder of the mixture 3 for 3 minutes.
5. Compress according to specifications.
6. Mix the solvent and the barrier against humidity for 45 minutes.
7. Coat with the aforementioned system according to specifications.

### Example 2. Pharmaceutical solid compositions

A pharmaceutical composition is prepared with 50 mg of tramadol hydrochloride and 75 mg of pregabalin by adding the following excipients (Table 1):

**Table 1**

| mg/tab | Components |
|---|---|
| 50.00 | Tramadol hydrochloride |
| 75.00 | Pregabalin |
| 349.50 | Microcrystalline cellulose |
| 7.70 | Aluminum metasilicate and magnesium |
| 12.80 | Sodium croscarmellose |
| 5.00 | Magnesium stearate |
| 20.00 | Opadry yellow |
| 0.082 | Purified water (mL) |

This solid pharmaceutical composition is in a solid dosage form, such as a tablet, a monolayer tablet, granules, caplets, lozenges, or pills. Most preferably, it is in tablet and/or pill form; ideally, the pharmaceutical composition with 50 mg of tramadol hydrochloride and 75 mg of pregabalin is in a biconvex tablet dosage form.

### Example 3 Pharmaceutical solid compositions

A pharmaceutical composition is prepared with 50 mg of tramadol hydrochloride and 150 mg of pregabalin by adding the following excipients (Table 2):

**Table 2**

| mg/tab | Components |
|---|---|
| 50.00 | Tramadol hydrochloride |
| 150.00 | Pregabalin |
| 455.50 | Microcrystalline cellulose |
| 7.70 | Aluminum metasilicate and magnesium |
| 12.80 | Sodium croscarmellose |
| 6.00 | Magnesium stearate |
| 18.00 | Opadry yellow |
| 0.075 | Purified water (mL) |

This solid pharmaceutical composition is in a solid dosage form, such as a tablet, a monolayer tablet, granules, caplets, lozenges, or pills. It is preferably in a tablet and/or pill form.

### Example 4. Pharmaceutical solid compositions

A pharmaceutical composition is prepared with 100 mg of tramadol hydrochloride and 75 mg of pregabalin by adding the following excipients (Table 3):

**Table 3**

| mg/tab | Components |
|---|---|
| 100.00 | Tramadol hydrochloride |
| 75.00 | Pregabalin |
| 398.50 | Microcrystalline cellulose |
| 7.70 | Aluminum metasilicate and magnesium |
| 12.80 | Sodium croscarmellose |
| 6.00 | Magnesium stearate |
| 20\|.00 | Opadry yellow |
| 0.075 | Purified water (mL) |

This solid pharmaceutical composition is in a solid dosage form, such as a tablet, a monolayer tablet, granules, caplets, lozenges, or pills. It is preferably in a tablet and/or pill form.

### Example 5 Stability testing

In accordance with current regulations, stability studies were carried out on three batches of the pharmaceutical compositions described in example 2 and 3. The results after 6 months under the condition of 40 °C and 75% relative humidity are shown below (Table 4 and 5):

**Table 4. 50 mg Tramadol / 75 mg Pregabalin**

| Determination | Specification | Result | | |
|---|---|---|---|---|
| | | Batch 1 | Batch 2 | Batch 3 |
| Tramadol Hydrochloride content | 90.0% to 110.0% 50.0 mg/tablet | 101.1% | 100.3% | 101.2% |
| Pregabalin content | 90.0% to 110.0% 50.0 mg/tablet | 100.6% | 99.3% | 101.3% |
| Tramadol Hydrochloride | Q = 80.0% in 30 minutes | 101.6% | 100.7% | 100.37% |

| Determination | Specification | Result | | |
|---|---|---|---|---|
| | | Batch 1 | Batch 2 | Batch 3 |
| Tramadol Hydrochloride content | 90.0% to 110.0% 50.0 mg/tablet | 99.1% | 99.3% | 99.2% |
| Pregabalin content | 90.0% to 110.0% 50.0 mg/tablet | 98.8% | 101.0% | 99.5% |
| Tramadol Hydrochloride content | Q = 80.0% in 30 minutes | 99.7% | 99.1% | 99.3% |
| Pregabalin content | Q = 80.0% in 30 minutes | 99.2\|% | 96.9% | 97.4% |

### Example 6. Bioavailability testing

The pharmacokinetic profile (Cₘₐₓ and AUC) of the single-dose 150 mg pregabalin / 50 mg tramadol combination versus each component administered individually in healthy subjects of both genders under fasting conditions was compared to establish the non-interaction of the combined drugs.

The pharmacokinetic parameters Cₘₐₓ, AUC, Tₘₐx, Ke, and T_{1/2} of combined pregabalin and tramadol after a single-dose oral administration were characterized: 150 mg pregabalin / 50 mg tramadol tablets (treatment C, Silanes Laboratories test drug) versus each component administered individually; 150 mg pregabalin capsules (treatment A, reference drug indicated by the regulatory authority) or 50 mg tramadol capsules (treatment B, reference drug indicated by the regulatory authority) in male and female healthy subjects under fasting conditions.

Frequency was established as a single dose after administration of a single dose of the formulations.

The study design was a crossover, 3x6x3, prospective, longitudinal single-dose study design of a 150 mg pregabalin / 50 mg tramadol combination administered orally versus each component administered individually, with three treatments, three periods, six sequences with a 7-day washout period, and with a number of 30 healthy subjects under fasting conditions.

Sampling times: 18 samples were collected from each research subject at the following times: 0.00 (pre-dose), 0.16, 0.33, 0.50, 1.00, 1.50, 2.0, 2.5, 3.0, 3.5, 4.0, 6.0, 10.0, 14.0, 24.0, and 36.0 hours at each of the three periods after a single-dose drug administration.

Analytical method: the analytical method for the plasma quantification of pregabalin and tramadol was based on the methods published by Patel et. al., J. Pharm. Biomed. 2009, 49(29), 354-66; Liu et. al., Eur. J. Drug Metab. Pharm. 2009, 34(3), 185-92; Vaidya et. al., Chromatographia 2007, 66(11), 925-8; and Mandal et. al., Chromatographia 2008, 67(8), 237-43, with some amendments. Blood samples from each period were received in glass tubes with sodium citrate as anticoagulant. The sample treatment technique was performed by precipitation; the separation technique was performed by HPLC using mass spectrometry detection. The analytical method complied with the validation parameters established in the Mexican standard NOM 177-SSA1-2013.

Statistical analysis: the results of the statistical testing applied to the pharmacokinetic parameters Cₘₐₓ, AUC₀₋ₜ, and AUC_{0-∞} obtained for the three treatments evaluated indicate that there is no difference in pharmacokinetic bioavailability between the oral formulations pregabalin and tramadol tablets versus each component administered individually; pregabalin capsules or tramadol capsules, in healthy subjects under fasting conditions.

The results obtained suggest the equivalence of the pharmacokinetic bioavailability of the three formulations analyzed. In addition, the results obtained allow concluding the non-interaction in the bioavailability of the formulations administered in combination.

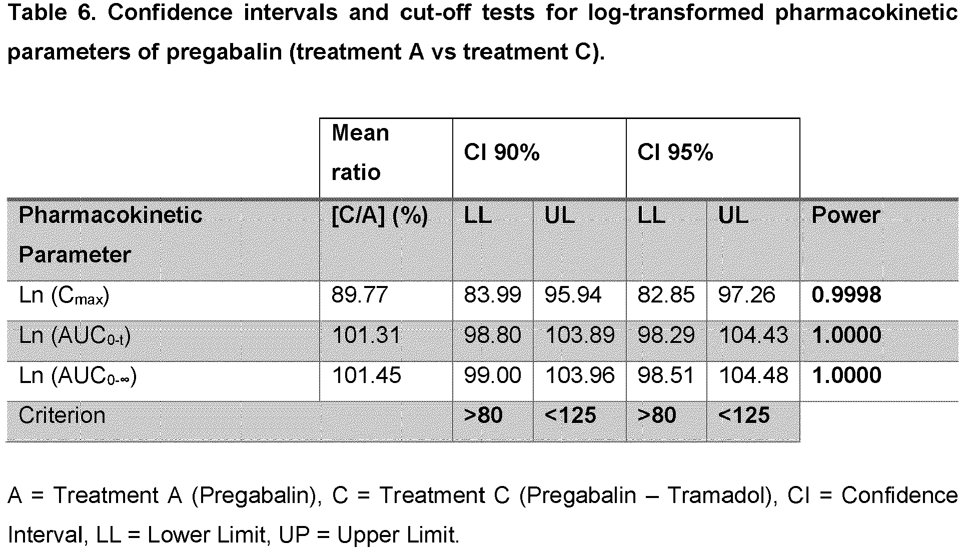

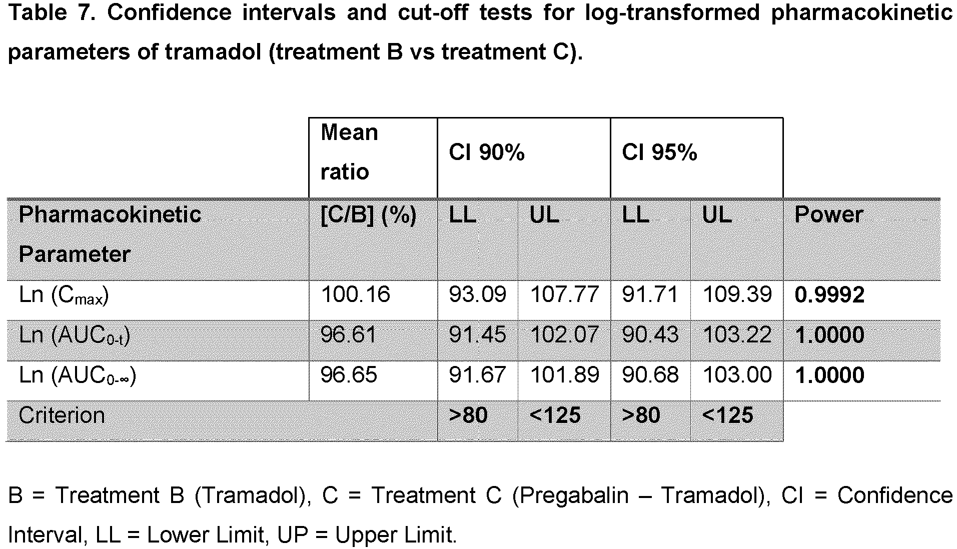

### Example 7. Dissolution testing

The dissolution test (*in vitro* test) is used to determine the rate (amount/time) and extent (total amount) at which a drug is released from the dosage form; the dissolution profile is the quantification at different times of the dissolved drug under standardized conditions. The importance of the dissolution test lies in the following:
a) It is a guide for the development of new formulations while developing a product: it enables the assessment of potential interference of excipients or the manufacturing process on drug release.
b) Process control and quality assurance: they help ensure the continuous quality of the product and its optimization after a change in manufacturing, formulation, manufacturing site, and process scale up.
c) *In vivo* development indicator: it is an indicator of bioavailability. It enables the correlation between *in vitro* parameters and bioavailability results to be established.

The dissolution profile testing was performed for tramadol hydrochloride / pregabalin 50/75 mg tablets and tramadol hydrochloride / pregabalin 50/150 mg tablets.

The quantification of pregabalin in the study was performed through a method previously validated under the criteria of the Mexican Official Standard NOM-177-SSAA-2013. The method was performed using a high performance liquid chromatography coupled to a UV-visible detector at a wavelength of 210 nm within the nominal concentration range of 16.6-99.6 mcg/mL and taking samples at 10, 15, 20, 30, and 45 min. The dissolution medium consisted of 0.06 N hydrochloric acid solution.

Similarly, the method for the quantification of tramadol hydrochloride was validated under the criteria of the Mexican Official Standard NOM-177-SSAA-2013. The method was performed using a high-performance liquid chromatography coupled to a UV-visible detector at a wavelength of 270 nm within the nominal concentration range of 11.1-66.6 mcg/mL and taking samples at 10, 15, 20, 25 and 30 min. The dissolution medium consisted of 0.1 N hydrochloric acid solution.

The percentages of tramadol hydrochloride and pregabalin in their respective dissolution medium for both the reference drug and the test drug were greater than 85% within the first 15 min, so these results show that the evaluated products can be accepted as equivalent (Figures 1 and 2).

Under the assessed conditions, the coefficients of variation of the percentages of the dissolved drugs were less than 20% at the first sampling time and less than 10% at subsequent sampling times for both tramadol hydrochloride and pregabalin for both drugs. The percentage of tramadol hydrochloride and pregabalin in their respective dissolution medium for both the reference drug and the test drug was greater than 85% within the first 15 minutes. These results show that the assessed products can be considered similar without the need to calculate the f2 similarity factor, as they dissolve very fast. The f2 similarity factor values reported were 96.2 for tramadol hydrochloride and 85.0 for pregabalin.

### Advantages of the invention and its industrial application

One of the main issues posed by drugs with synergistic compositions of known drugs and known concentration is the difficulty in ensuring that their stability, dissolution, and bioavailability are maintained once administered to the patient, especially when the drugs are highly sensitive to light and humidity. In this regard, despite being within the normative parameters of dissolution and bioavailability, the results often tend to be close to the lower limit of the specification, resulting in a delay in the therapeutic effect or a higher dose being required.

This invention, which involves a stable, immediate-release combination of pharmaceutical compositions, i.e., an opioid analgesic drug such as tramadol with an antiepileptic/analgesic drug such as pregabalin, is useful for the treatment and control of pain, such as neuropathic pain and/or acute neuropathic pain, since the synergistic effect of the drugs is maintained at low doses while maintaining the dissolution and bioavailability of the composition without losing its therapeutic effect once administered, as shown by the pharmacokinetic bioavailability after the administration of the composition of this invention and the reference oral formulations.

## Claims

1. This is a solid, coated, stable pharmaceutical composition against pain, neuropathic pain, and/or acute neuropathic pain comprising tramadol or a pharmaceutically acceptable salt thereof in concentrations between 50-150 mg in combination with pregabalin or a pharmaceutically acceptable salt thereof in concentrations of 50-150 mg; a light-protective coating system that enhances stability, without altering dissolution and bioavailability; and at least one pharmaceutically acceptable excipient.

2. As per claim 1, the pharmaceutical composition is **characterized by** the fact that the concentration of tramadol or a pharmaceutically acceptable salt thereof should preferably be between 50-100 mg, and the concentration of pregabalin or a pharmaceutically acceptable salt thereof should preferably be between 75 to 150 mg.

3. As per claims 1 and 2, the pharmaceutical composition is **characterized by** the pharmaceutically acceptable excipient being selected from one or more diluents, disintegrants, lubricants, and absorbents.

4. As per all of the preceding claims, the pharmaceutical composition is **characterized by** a pharmaceutically acceptable diluent selected among microcrystalline cellulose, phosphate derivatives such as dibasic calcium phosphate, starch derivatives such as pregelatinized starch and corn starch, as well as mannitol, xylitol, maltitol, lactitol, sorbitol, sucrose, or a combination thereof.

5. As per claim 4, the pharmaceutical composition is **characterized by** the diluent being present preferably in a concentration of 5 to 90 wt%.

6. As per claims 1 to 3, the pharmaceutical composition is **characterized by** the disintegrant being selected among croscarmellose; cellulose derivatives such as hydroxypropyl cellulose, carboxymethyl cellulose, and microcrystalline cellulose; povidone derivatives such as crospovidone; and starch derivatives such as pregelatinized starch, sodium starch glycolate, or corn starch.

7. As per claim 6, the pharmaceutical composition is **characterized by** the fact that the disintegrant may be present in an amount ranging from 0.5 to 15 wt%, preferably between 0.5 to 3 wt%.

8. As per claims 1 to 3, the pharmaceutical composition is **characterized by** a lubricant selected among magnesium stearate, zinc stearate, calcium stearate, stearic acid, monostearate, stearyl fumarate, talc, or sulfated derivatives such as magnesium lauryl sulfate.

9. As per claim 8, the pharmaceutical composition is **characterized by** the lubricant being present in an amount ranging from 0.25 to 10 wt%, preferably from 0.8 to 1 wt%.

10. As per claims 1 to 3, the pharmaceutical composition is **characterized by** an absorbent selected among aluminum derivatives (aluminum hydroxide, aluminum oxide, aluminum phosphate), clay or soil derivatives (attalpugite, bentonite, hectorite, kaolin, pectin), silica derivatives (calcium silicate, colloidal silicon dioxide, magnesium aluminum silicate), cellulose derivatives (microcrystalline cellulose, cellulose), or magnesium derivatives (magnesium carbonate, magnesium silicate).

11. As per claim 10, the pharmaceutical composition is **characterized by** the fact that the absorbent is present in an amount ranging from 0.5 to 90 wt%, preferably from 1 to 3 wt%.

12. As per claim 1 or 2, the pharmaceutical composition is **characterized by** a coating system that is a cellulose derivative selected among hydroxypropyl methylcellulose; hydroxypropyl cellulose; carboxymethylcelluloses; polyvinyl derivatives such as polyvinyl alcohol; polyethylene glycol; and povidones in all K values, as well as their derivatives.

13. As per claim 12, the pharmaceutical composition is **characterized by** the fact that the coating system is present in a concentration ranging from 0.5 to 6 wt%, preferably between 2.5 to 3.5 wt%.

14. As per all of the preceding claims, the pharmaceutical is **characterized by** being a solid pharmaceutical composition.

15. As per claim 14, the pharmaceutical composition is **characterized by** being in the form of a tablet, pill, caplet, granules, or capsules.

16. As per claim 15, the pharmaceutical composition is **characterized by** being preferably in the form of a tablet and/or pill.

17. As per claim 15, the pharmaceutical composition is **characterized by** being preferably designed as a caplet with a breakline.

18. As per claim 16, the pharmaceutical composition is **characterized by** being preferably in the form of a biconvex tablet.

19. As per claims 1 to 18, the pharmaceutical composition is used for the manufacture of a drug product suitable for the treatment of pain.

20. As per claim 19, the pharmaceutical composition is used against neuropathic pain.

21. As per claim 20, the pharmaceutical composition is used against acute neuropathic pain.
